# EUROPEAN PATENT SPECIFICATION

(11) **EP 1 707 065 B1**
(45) Date of publication and mention of the grant of the patent: **29.06.2016**
(21) Application number: 06006532.3
(22) Date of filing: 29.03.2006
(51) Int. Cl.: A43B 3/00, A61B 5/103, A61B 5/11, G01C 22/00

(54) **POD for mounting electronic device to footwear**
Gehäuse zum Montieren von elektronischen Geräten an Schuhwerk
Boîtier pour le montage d'appareils électroniques sur un article chaussant

(30) Priority: 31.03.2005 DE 102005014709
(43) Date of publication of application: 04.10.2006
(60) Divisional application: 10179293.5
(73) Proprietor: adidas International Marketing B.V., 1062 KR Amsterdam (NL)
(72) Inventor: Carnes, James, Portland Oregon 97201 (US); Vincent, Stephan Michael, Portland Oregon 97229 (US); Tomlinson, Scott, Portland Oregon 97210 (US)
(74) Representative: Wegner, Hans

(56) References cited:
- EP-A- 0 121 026
- WO-A-95/26652
- GB-A- 2 300 559
- US-A- 4 703 445
- US-A- 4 891 797
- US-A- 5 921 008
- US-A- 6 017 128
- US-A1- 2003 000 053
- US-B1- 6 356 856
- US-B1- 6 560 903
- US-B1- 6 788 200

## Description

### 1. Technical field

The present invention relates to a shoe for receiving a housing of an electronic assembly.

### 2. The prior art

In an increasing number of sports professional athletes as well as amateurs desire to electronically keep track of their athletic performance. For example electronic speedometers with a plurality of functions are nowadays the standard equipment of every ambitious race or mountain biker. Small computers are also used for running which count the number of steps, the speed, and thus the covered distance and provide further information.

Whereas it is comparatively easy to mount a bicycle computer, there exists a problem in the case of running. On the one hand the small computer should somehow be connected to the shoe in order to reliably detect the steps of the athlete, using for example a contact sensor or a shock sensor or accelerometer in the shoe sole. On the other hand, the small computer must be protected against humidity and mechanical damage. Finally, the small computer should be easily accessible to view the recorded data and to perform inputs, if desired, or to connect the small computer to a PC for further evaluating the measured data.

A similar problem occurs if a runner intends to carry other types of small computers, such as a MP3-Player. Although it is generally possible to attach such an electronic assembly to the body using a strap or the like, such an attachment is in many cases considered to be uncomfortable during a high performance of the body.

Therefore, many approaches are known from the prior art as to how to attach small computers to a shoe. For example the US 6,536,139, the US 6,278,378 and the JP 60 200120 disclose arrangements, wherein the small computer is attached to the laces or the tongue of a shoe. A modification of this design is disclosed in the US 4,649,552, the JP 60 84689, the US 6,183,425 and the WO 88/04768, wherein a sensor is integrated into the sole region. The small computer, which processes the signals of the sensor to provide useful data, is again arranged in the region of the laces and/or on the instep. The US 4,771,394 discloses in addition a system, wherein the small computer is attached to the heel of a shoe.

WO 95/26652 discloses a shoe comprising a sole unit, the sole unit comprising a recess for receiving a housing of an electronic assembly, the recess comprising a projection engaging a corresponding indentation of the housing.

From EP 0 121 026 A1 an illuminated sport shoe is known. A cavity is defined in an integral sole of the shoe and a battery is mounted in the cavity.

Further, GB 2300559 A relates to sole with a chamber for receiving a battery.

US 6,017,128 relates to footwear with flashing lights. In one embodiment, a module is described which can be plugged into a cavity formed in an upper surface of a mid-sole in a vertical direction.

US 6,788,200 relates to footwear with GPS. In one embodiment, a transponder is located in the midsole of the footwear.

Finally, US 4,703,445 discloses an athletic shoe for running disciplines. In particular, in an area of the sole of the shoe that is less stressed during use, at least one free space is provided where a transmitter is housed which, via a sensor provided in the sole, can emit at least one output signal.

However, all of these prior art arrangements of a small computer on the shoe have the disadvantage that the sensitive electronic components of the small computer are only insufficiently protected against damage. When the runner stumbles or falls, there is a significant risk that the housing of the small computer, which is typically made from plastic materials, is subjected to excessive mechanical loads. Protective coatings, which are sufficiently effective, are excluded, since the electronic assembly would reach a size and weight impairing the course of motion, when the electronic assembly is arranged on the outside of the shoe.

The present invention is therefore based on the problem to provide a shoe allowing to securely receive an electronic assembly, such as an electronic pedometer, an accelerometer or speed sensor, in order to overcome the above discussed disadvantages of the prior art.

### 3. Summary of the invention

The invention is defined in independent claim 1.

The invention allows to releasably integrate not only a sensor but a complete electronic assembly, such as a pedometer, an accelerometer or speed sensor, a MP3-Player or any arbitrary electronic assembly into the shoe sole. Using a cable or a wireless transmission an additional user interface may be connected to the electronic assembly to allow inputs or the reading of measured data even during operation.

The present invention is based on the realization that the required circuits for electronic assemblies have become smaller and smaller and can therefore easily be arranged in a recess of the sole of a shoe without significantly affecting the functional properties of the sole (cushioning and support). At the same time the electronic assembly is completely enclosed and therefore perfectly protected against mechanical impacts. An arrangement in a recess of the sole is also aesthetically pleasing, since the housing of the electronic assembly can preferably not be recognized from the outside.

Since the housing is not permanently integrated into the sole but only releasably arranged in the recess, it is always possible to replace the housing, for example to exchange a faulty electronic assembly, to exchange the battery or to remove the housing and its electronic assembly from the recess so that it can be connected to a PC or the like for a further evaluation of the data.

Finally, the detachable arrangement of the housing also makes it easy to replace one assembly by another one to realize a different functionality. A unit for detection of the running movement by counting steps or measuring accelerations can thus be immediately exchanged against another assembly, e.g. a GPS receiver for the determination of a position, a pulse sensor or other electronic function units. The exchangeability is particularly advantageous in the context of an intelligent shoe, wherein the cushioning properties of the sole can be changed under the control of an electronic control unit. If this control unit is defective, to be provided with a new firmware or to be exchanged against a more recent model, the present invention can be used in a particularly advantageous manner. It is also perceivable for the assembly to be removed in order to exchange, read, expand etc. the memory of the electronic assembly.

The recess is arranged in the midfoot region of the sole unit. Thus, the housing is arranged in a region of the sole which is subjected to the smallest ground reaction forces and accordingly subjected to the smallest deformations.

In one embodiment the sole unit has an opening and/or a window to view and/or to operate the electronic assembly. In this version an additional user interface is not required but also not excluded.

The recess is arranged in a midsole of the sole unit which is covered by a removable insole, when the shoe is used. If the housing for an electronic assembly is arranged in such a recess, it is well protected, since it is preferably surrounded by sole material from all sides. The insole is removable and therefore does not impair the direct access to the housing of the electronic assembly, for example for changing the battery.

In a presently particularly preferred embodiment, the recess has a shape corresponding to the housing of the electronic assembly. This leads to a positive fit when the housing is inserted so that the housing is arranged essentially without play inside the recess of the sole. Vibrations, which after some time could damage an electronic assembly inside the housing, are therefore essentially excluded.

In a first embodiment, which is not part of the invention the recess comprises at least one indentation or opening. Particularly preferred are two, essentially opposing indentations or openings which can be engaged by one or two projections, respectively, of the housing. The recess comprises preferably at least one elastic element, preferably an elastic boundary surface, in order to rigidly fix the housing inside the recess. As a result, the housing is in addition to the explained positive fit actively anchored to the recess so that almost any movement of the housing inside the recess is avoided.

The recess comprises at least one projection engaging a corresponding indentation or opening of the housing. Preferably, the recess comprises at least one, preferably two projections both on the front end and on the rear end of the recess in the shoe's longitudinal direction, each engaging corresponding indentations or openings of the housing.

Further, a removal strap is preferably arranged in the recess to facilitate the removal of the housing from the recess. The removal strap is preferably arranged on the recess such that it extends below the assembly arranged in the recess and that one end of the strap extends over the upper edge of the recess. In one embodiment, the removal strap further comprises a section which is engaged by a projection of the housing when it is arranged inside the shoe's recess.

The recess is integrated into a reinforcing element of the sole unit, in particular a support bar which allows torsional flex of the foot i.e. a rotation of the forefoot part relative to the rear foot part. Such a torsion support or torsion bar is typically made from an essentially incompressible material to achieve the desired support function and is therefore particularly suitable for securely receiving the housing for the electronic assembly. Further, tests have shown that such an assembly leads to the most exact measuring results if an accelerometer is used.

A housing is disclosed for receiving an electronic assembly, in particular a pedometer, an accelerometer or a speed sensor, wherein the housing has an outer shape corresponding to the shape of a receptacle in a shoe. Such a housing is particularly suited to be used together with a shoe having the above described features and allows to securely receive an electronic assembly, such as a pedometer, an accelerometer or a speed sensor, an MP3-Player, a combination of the two assemblies or an electronic assembly having other functions, such as an electronic assembly for measuring the pulse. The housing may be permanently connected to the electronic assembly or the electronic assembly is releasably arranged inside the housing.

Preferably, the housing comprises a coating of an elastic material, which substantially fully encompasses the housing. Thus, a firm fit in the shoe's recess is on the one hand obtained and, on the other hand, the housing is protected, when it is removed from the recess and e.g. falls down onto a hard floor.

In a preferred embodiment the housing comprises an attachment cover which can be releasably attached to the housing and which at least partially surrounds the housing. The attachment cover includes preferably an attachment strap which is provided to extend below the laces or other attachment means of a shoe. The attachment strap has preferably a free end and the housing comprises a means for releasably attaching the free end of the attachment strap.

Using the preferred attachment cover, the described housing may also be releasably attached in a conventional manner to any arbitrary shoe, if this shoe does not provide a suitable recess in the shoe sole. The use of the housing and the electronic assembly arranged therein does therefore not necessarily require the use of a unique pair of shoes. Moreover, the mounting cover makes it possible to use the assembly additionally or alternatively for a display or the like, which is arranged on the laces and displays data measured and/or generated by a further assembly within the shoe.

Further modifications of the invention are the subject matter of further dependent claims.

### 4. Short description of the drawings

In the following detailed description a presently preferred embodiment of the invention is described with reference to the drawings. The drawing comprises the following figures:
- Fig. 1a, b;: a schematic representation of the steps which are required for arranging the housing in a sole unit of a shoe according to a first embodiment of the present invention;
- Fig.2:: a schematic representation how the housing is inserted into the recess of the sole unit;
- Fig. 3:: a detailed representation of the projections of the housing which are coated by a rubber layer;
- Fig. 4:: a detailed representation of a section along the line A - A in Fig. 3, when the projection is inserted into an opening of the recess;
- Fig. 5:: an exploded representation of the housing in a first embodiment;
- Fig. 6:: a representation of the attachment cover for the housing in a first embodiment;
- Fig. 7, 8:: schematic representation showing how the housing is attached to the laces of a shoe;
- Fig. 9: a schematic drawing as to how the housing may be arranged in a sole unit in accordance with another embodiment of the present invention;
- Fig. 10:: a detailed illustration of the removal strap in the embodiment of Fig. 9 to facilitate removal of the housing from the recess;
- Fig. 11a:: a detailed illustration of a mounting cover for the housing in another embodiment;
- Fig. 11b:: a detailed illustration of the housing in another embodiment, adapted to the mounting cover of Fig. 11a; and
- Fig. 12:: a schematic representation showing how an electronic assembly can communicate in a wireless manner with a user interface provided on a receiving unit attached to a user.

### 5. Detailed description of preferred embodiments

In the following a presently preferred embodiment of the present invention is further described with reference to an electronic pedometer in a running shoe. In the following the term "pedometer" comprises any electronic assembly detecting in any electronic manner the running movement, e.g. to determine the number of steps done, the acceleration, the speed or other physical values in connection with the running movement. However, it is to be understood that the present invention also relates to the arrangement of other kinds of small electronic assemblies in shoes, as they were exemplary and in a non-limiting manner mentioned above.

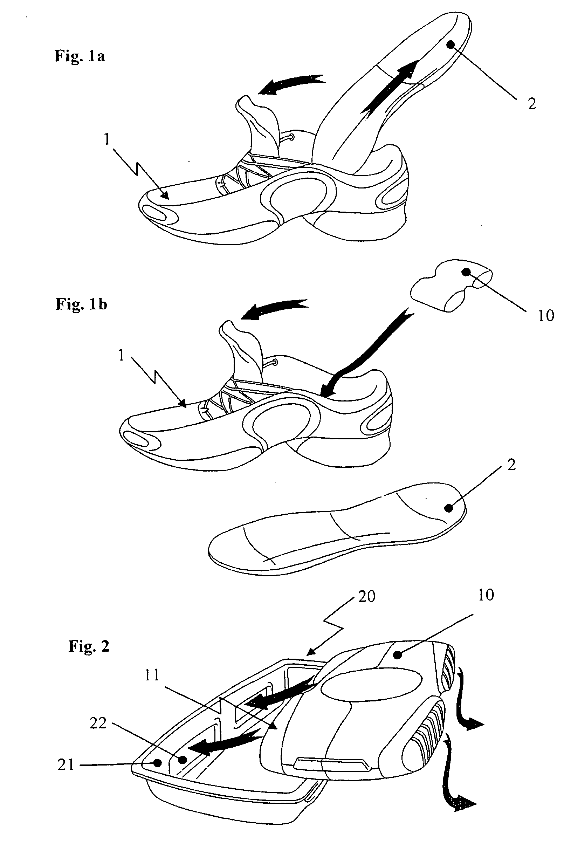

Figures 1a and 1b schematically show how an electronic pedometer (not shown) inside a housing 10 is arranged in a recess of a sole of a shoe 1. To this end, a removable insole 2 or a so-called sock liner is at first removed (cf. Fig. 1a). Subsequently, the housing 10 is arranged in a recess of the midsole (cf. Fig. 1b) which is not shown in figs. 1a and 1b.

In another embodiment, which is not shown, there may even be inputs to the electronic assembly 10 after it has been arranged in the midsole. To this end, openings (not shown) are arranged in the surrounding sole layers or keys are provided which allow the operation of corresponding input means on the housing 10 (not shown). In this context, it is also conceivable to manufacture parts of the sole layers surrounding the inserted housing 10 from a transparent material to allow an inspection of a display on the housing or to allow the transmission of a light signal which might, for example, indicate the proper function of the electronic assembly. In this embodiment, the housing itself preferably also comprises a window and means for operating keys of the electronic assembly, if required, or for observing a display.

An exemplary illustration of the recess 20 in the midsole is shown in Fig. 2. For the sake of simplicity, the midsole material surrounding the recess 20 is not shown. The recess 20 has essentially the shape of a tub, wherein at least one of its inner dimensions, i.e. its length, width or depth corresponds essentially to the outer dimensions of the housing 10. Preferred, however, is a correspondence of all dimensions. When the housing 10 is inserted into the recess 20, as shown in Fig. 2, there is a positive fit so that relative movements inside the recess 20 are essentially excluded. Furthermore, the top surface of the inserted housing 10 is preferably flat to avoid bulges in the insole 2. Therefore, such an arrangement of the housing 10 has no significant influence on the wearing comfort of the shoe 1. The recess 20 is arranged in the midfoot area of the shoe and for example integrated into a device for supporting the arch of the foot, which is typically made from a harder material than the surrounding sole material. Particularly preferred is an arrangement of the recess 20 in a support bar which allows torsional flex of the foot i.e. rotation of the forefoot relative to the rear foot, as it is used in sports shoes of applicant.

Figure 2 discloses an attachment alternative, which is particularly easy to realize and which is not part of the invention. To this end, openings 22 are arranged in the sidewalls 21 of the recess 20. Projections 11 of the housing 10 extend through the openings and thereby attach the housing 10 inside the recess 20. As can be seen from Fig. 2, the sidewalls 21 preferably comprise several openings 22, which are preferably arranged in pairs on opposing sidewalls 21. The sidewalls 21 may be the longitudinal sides and/or the lateral sides of the recess 20.

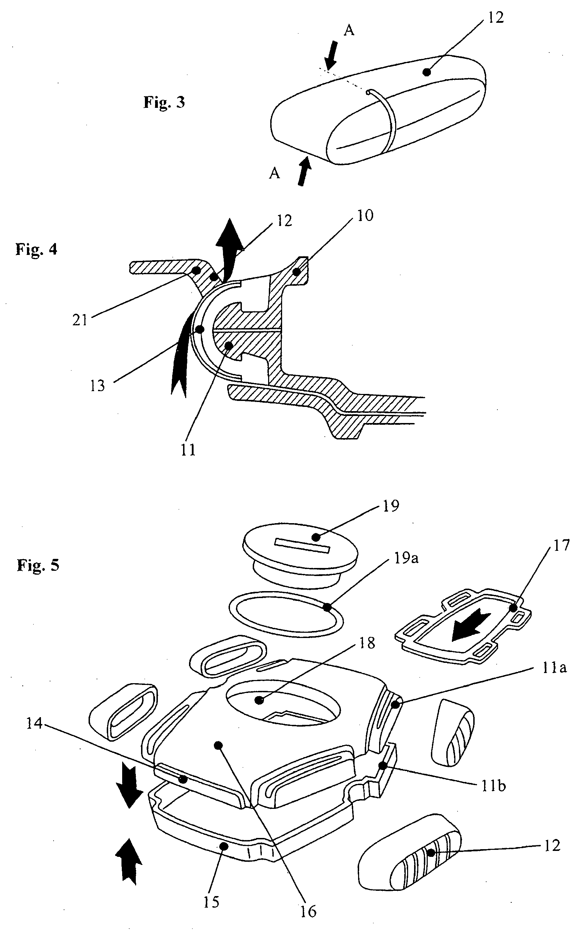

The arrows in Fig. 2 illustrate how the projections 11 are initially inserted into the corresponding openings 22 of a sidewall 21. Subsequently, the housing 10 is downwardly pressed so that also the opposite projections 11 can engage corresponding openings or indentations 22 of the opposite sidewall. To this end, it is necessary that the sidewalls 21 and/or the projections 11 have a certain elasticity. In the embodiment shown in the figures, the projections 11 have an outer rubber coating 12 providing the required elasticity. The rubber coating 12 may either be made from a continuous rubber material or it comprises inner cavities (cf. Fig. 4) filled by air or, if necessary, specific cushioning materials such as a gel (not shown). For an easier handling of the housing 10, the outside of the rubber coating 12 comprises a profile (cf. Fig. 3).

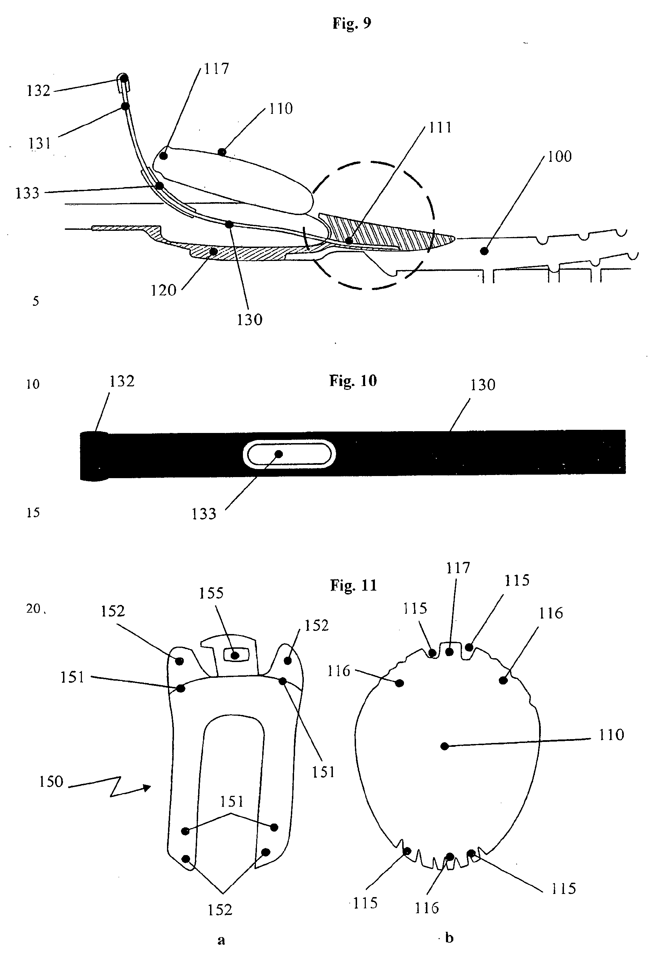

The rubber coating 12 additionally protects the housing 10 and its electronic components of the pedometer when the housing 10 is removed, e.g. for connecting to a PC, and unintentionally dropped. It is therefore also conceivable to provide the housing 10 with a rubber coating which covers not only the disclosed four projections 11 but which completely covers the housing 10 as explained below in more detail with reference to the further embodiment shown in Fig. 11b. This is particularly advantageous, when an attachment cover is used, as described below, so that the housing 10 is arranged on the laces without the protection of the surrounding sole material.

As already mentioned, the tub 20 may also comprise elastic inserts or edge sections (not shown) to ensure a firm fit of the housing 10 inside the recess 20.

If the electronic pedometer uses a shock sensor, it is additionally advantageous to anchor the housing 10 in the recess 20 without any play. Any acceleration acting on the housing 10 and thereby on the pedometer reflects an acceleration of the shoe 1 and not to a relative movement between the housing 10 and the shoe 1.

Figure 5 shows an exploded view of the housing 10 in an embodiment which is not part of the invention. As can be seen, the presently preferred construction comprises a lower half 15 and an upper half 16 with an intermediate sealing 17 to avoid the penetration of humidity. Such a sealing 17 is particularly preferred, when the two halves 15 and 16 are releasably interconnected to allow the removal of an electronic assembly (not shown) arranged inside the housing 10. Alternatively, it is also conceivable to permanently interconnect the two halves 15, 16 after the initial installation of the electronic assembly, for example by gluing, high-frequency welding or other techniques.

In the embodiment of Fig. 5, two halves 15, 16 are interconnected by having an integral rubber coating 12 extending over both, the upper half 11 a and the lower half 11b of a lateral projection of the housing. Additionally or alternatively, latches and/or screws can be used for interconnecting the upper and the lower half of the housing. Finally, the housing 10 may also be provided as a single component.

As shown in Fig. 5, the disclosed embodiment preferably comprises an opening 18 on its upper side with a cover 19 and an assigned sealing 19a, for example an O-ring. As a result, a battery or an accumulator can be easily replaced, without the housing 10 having to be removed from the recess 20. Instead of an opening, the housing 10 could also comprise a charging socket so that an accumulator arranged in its interior can be charged using a cable (not shown).

Figures 9, 10 and 11b show a further, presently preferred embodiment of the present invention. To this end, an approximately tub-like recess 120 is arranged in the midfoot region of a shoe midsole, the inner dimensions of which correspond substantially to the outer dimensions of a corresponding housing 110. As shown in Fig. 11b, the housing 110 has preferably an approximately turtle-like shape.

In a lateral region, e.g. at the front end of the recess 120 which is marked in Fig. 9 by a dashed circle, there is a slit 111 in which a removal strap 130 is mounted, e.g. glued. When the housing 110 is inserted into the recess 120, as shown in Fig. 9, the removal strap 130 is flat below the housing 110. Only a boundary region 131, preferably comprising a reinforcing element 132 to facilitate gripping, protrudes from the recess 120. Since, however, the boundary region 131 and the reinforcing element 132 are preferably arranged below an insole, i.e. a so-called sock liner (not shown in Fig. 9), the comfort of wearing the shoe is not impaired.

The removal strap 130 facilitates removal of the housing 110 of the electronic assembly from the shoe sole. Despite a firm fit in the recess 120, as obtained e.g. by covering the housing 110 with an elastic layer, e.g. a rubber layer, the housing 110 may be removed from the recess 120 of the sole simply by pulling the removal strap. Neither a particular force nor specific tools are required.

Fig. 10 shows a preferred embodiment of the removal strap 130. As can be directly seen, the removal strap of this embodiment has a cutout 133 engaged by a projection 117 of the housing 110 (cf. Fig. 11b) in the mounted position of the housing 110 in the recess 120. Thus, the removal strap 130 is correctly aligned to facilitate the later removal of the housing 110 in the manner explained above.

Fig. 11b further shows that the housing 110 in the presently preferred embodiment comprises a plurality of indentations 115, which are preferably arranged in pairs at the front and rear ends of the housing 110. Projections, which are correspondingly arranged in the interior of the recess 120, preferably in pairs at the front and rear ends of the recess 120 (not shown in the section of Fig. 9) engage these indentations. Thus, the housing 110 is firmly positioned inside the recess 120.

For the better handling of the housing and the electronic assembly with its sensitive circuits, it is advantageous for the rubber layer to have regions 116 with groove-like indentations, facilitating a secure grip. The embodiment of Fig. 11b shows three of such regions, namely in the front besides the above-explained preferred indentations 115 and in the rear between the two rear indentations, respectively.

Finally, also measures other than the interaction of the explained projections and indentations are perceivable to ensure a secure mounting of the housing in the recess. For example, the housing may be fixed in a certain position by a magnet (not shown), or the recess comprises a closable cover which additionally fixes the removable housing (not shown).

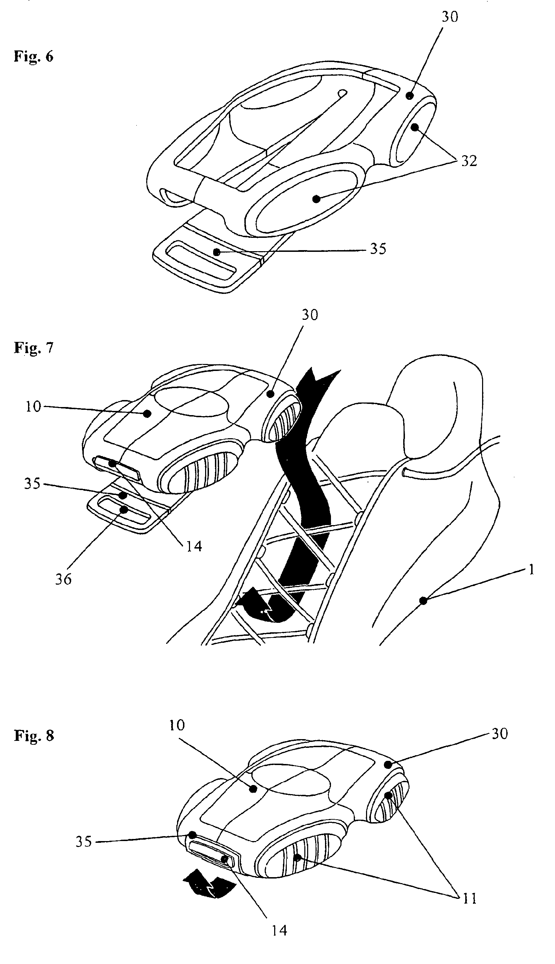

Figs. 6 to 8 illustrate an embodiment of an attachment cover 30, which is also part of the present invention. The attachment cover 30 allows to attach the above-described housing 10 to the laces of a common shoe, if a further electronic assembly, e.g. an MP3 player is to be arranged on the shoe in addition to a first electronic assembly, e.g. a pedometer. Similar to the recess 20, the attachment cover 30 comprises sidewalls with openings 32, which can be penetrated by the projections 11 of the housing 10. As a result, the housing 10 can be reliably but releasably fixed to the attachment cover 30. Also here the openings 32 are preferably arranged in opposite pairs. The preferred embodiment comprises altogether four openings 32 similar to the above-explained embodiment of the recess 20, which are penetrated by four corresponding projections 11 of the housing 10 (cf. Fig. 8).

On its lower side, the attachment cover 30 preferably comprises an attachment strap 35, serving for a removable connection to the laces (cf. Fig. 7) or to other closing means of a shoe (not shown). The attachment strap 35 is guided below the laces. At its free end, its comprises an opening 36 so that the free end can be releasably attached to the front end of the housing 10 (cf. Fig. 8). To this end, a suitable projection 14 is arranged aon the housing 10. Other attachment means are also conceivable, for example a snap-fastener for securely connecting the free end of the attachment strap 35 to the housing 10 and/or the attachment cover 30.

An alternative embodiment of the mounting cover 150, which is preferably used with the embodiment of the housing from Fig. 11b, is shown in Fig. 11a. The mounting cover 150 only partially encompasses the housing 110. To this end, it comprises arms 151 extending when in use below the laces (not shown) and ending in hook-like end regions 152, being upwardly bent. The housing 110 is clipped into the resulting receptacle from above so that the hook-like end regions 152 encompass the housing at its top surface. Instead of the four arms 151 shown in Fig. 11a, other numbers of arms are possible, e.g. three arms or only two arms of wider dimensions, encompassing the housing 110 at the top and bottom end or at the sides.

Further, a mounting flap 155 may be arranged on the mounting cover 150, as shown in Fig. 11a, extending over the projection 117 and thus anchoring the housing 110 securely in the mounting cover 150 in addition to clipping into the hook-like end regions 152.

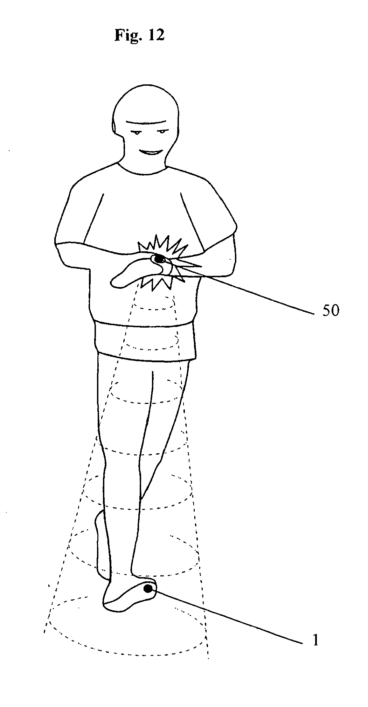

Fig. 12 discloses a further option for controlling the electronic assembly arranged inside the sole or on the instep in addition or alternatively to the above described input possibilities through the sole. Using a wireless link, the electronic assembly arranged in the housing 10 inside the shoe sole communicates with a receiving unit 50 carried on the wrist. To this end, a wireless transmission standard such as Bluetooth® or the like may be used. Since the actual electronics are arranged inside the housing in the shoe sole, the receiving unit 50 at the wrist (or at any other position) can be very light-weight, small and for example be integrated into a watch or garment. However, an arrangement in a strap similar to an electronic assembly for measuring the pulse is also conceivable.

The wireless transmission between the unit 50 and the electronic assembly inside or at the shoe sole may be bi-directional so that the electronic assembly can be controlled using the unit 50. Instead of a wireless transmission, a thin cable may be used which is guided through the housing 10 to the outside and connected to a control and / or display unit outside the shoe sole (not shown).

Moreover, the electronic assembly may be electrically connected to a plurality of additional sensors which are not arranged in the housing itself but at other locations inside the shoe, e.g. in the sole region, in the shoe upper, in the forefoot region or in the rearfoot region etc. Since not all possible connections to the electronic assembly are required for every shoe, it is also perceivable that only some of a plurality of electrical connections are actually connected. This is particularly reasonable, if a vendor distributes an electronic assembly which is able to detect a large number of different parameters (speed, acceleration, GPS position, deformation of the shoe, pulse rate etc.) during running, which are provided by a complex and thus expensive shoe with the entirety of all possible sensors. Due to its modular character, the device can, however, also be used with a simpler and cheaper shoe comprising only some of the sensors.

## Claims

1. Shoe (1), in particular sports shoe, comprising a sole unit, wherein the sole unit comprises a recess (20, 120) for releasably receiving a housing (110) of an electronic assembly, in particular an electronic pedometer, wherein
a. the recess (20, 120) is arranged in the midsole of a sole unit which is covered by a preferably removable insole, when the shoe is in use,
b. the recess (20, 120) is arranged in the midfoot region of the sole unit,
c. the recess (120) comprises at least one projection engaging a corresponding indentation or opening of the housing (110),
d. the recess (20) is integrated into a reinforcing element of the shoe sole.

2. Shoe (1) according to one of the preceding claims, wherein the sole unit comprises an opening and / or a window to inspect and / or to operate the electronic assembly.

3. Shoe (1) according to one of the preceding claims, wherein the recess (20, 120) has a shape corresponding to the shape of the housing (10, 110) of the electronic assembly.

4. Shoe (1) according to the preceding claim, wherein the recess (20) comprises at least one indentation or opening (22) to be engaged by a projection (11) of the housing (10).

5. Shoe (1) according to the preceding claim, wherein the recess (20) comprises at least two indentations or openings (22) which are substantially opposite to each other.

6. Shoe (1) according to one of the preceding claims, wherein the recess (20) comprises at least one elastic element, preferably an elastic boundary surface to firmly attach the housing (10) in the recess (20).

7. Shoe (1) according to the preceding claim, wherein the recess (120) comprises one, preferably two projections at the front end respectively rear end of the recess, seen in the shoe's longitudinal direction, each engaging corresponding indentations or openings of the housing (110).

8. Shoe (1) according to one of the preceding claims, wherein a removal strap (130) is further arranged in the recess (120) to facilitate removal of the housing (110) from the recess (120).

9. Shoe (1) according to the preceding claim, wherein the removal strap (130) is arranged in the recess (120) such that it is positioned below the housing (110) arranged in the recess and that an end (131, 132) of the strap (130) extends over the top edge of the recess (120).

10. Shoe (1) according to one of the two preceding claims, wherein the removal strap (130) comprises a cut-out (133) engaged by a projection (117) of the housing (110), when the housing is arranged inside the recess (120) of the shoe.

11. Shoe (1) according to claim 1, wherein the reinforcing element comprises a support bar, which preferably allows torsional flex of the foot, i.e. rotation of the forefoot relative to the rearfoot.

12. Shoe according to one of the preceding claims, wherein the shoe further comprises an attachment cover (30, 150), engaging the laces or other attachment means of the shoe, and in which a second electronic assembly may be arranged.

## Patentansprüche

1. Ein Schuh (1), insbesondere ein Sportschuh, umfassend eine Sohleneinheit, wobei die Sohleneinheit eine Aussparung (20, 120) zum lösbaren Aufnehmen eines Gehäuses (110) einer elektronischen Baugruppe umfasst, insbesondere eines elektronischen Schrittzählers, wobei
a. die Aussparung (20, 120) in der Mittelsohle einer Sohleneinheit angeordnet ist, welche durch eine vorzugsweise abnehmbare Innensohle bedeckt ist, wenn der Schuh in Benutzung ist,
b. die Aussparung (20, 120) im Mittelfußbereich der Sohleneinheit angeordnet ist,
c. die Aussparung (120) zumindest einen Vorsprung umfasst, welcher an eine entsprechenden Vertiefung oder Öffnung des Gehäuses (110) koppelt,
d. der Vorsprung (20) in einem Verstärkungselement der Schuhsohle integriert ist.

2. Schuh (1) nach einem der vorherigen Ansprüche, wobei die Sohleneinheit eine Öffnung und/oder ein Fenster umfasst, um die elektronische Baugruppe zu kontrollieren und/oder zu betreiben.

3. Schuh (1) nach einem der vorherigen Ansprüche, wobei die Aussparung (20, 120) eine Form hat entsprechend zu der Form des Gehäuses (10, 110) der elektronischen Baugruppe.

4. Schuh (1) nach dem vorherigen Anspruch, wobei die Aussparung (20) zumindest eine Vertiefung oder Öffnung (22) umfasst, um durch einen Vorsprung (11) des Gehäuses (10) angekoppelt zu sein.

5. Schuh (1) nach dem vorherigen Anspruch, wobei die Aussparung (20) zumindest zwei Vertiefungen oder Öffnungen (22) umfasst, welche sich im Wesentlichen gegenüber liegen.

6. Schuh (1) nach einem der vorherigen Ansprüche, wobei die Aussparung (20) zumindest ein elastisches Element umfasst, vorzugsweise eine elastische Begrenzungsfläche, um das Gehäuse (10) fest in der Aussparung (20) zu befestigen.

7. Schuh (1) nach dem vorherigen Anspruch, wobei die Aussparung (120) eine, vorzugsweise zwei Vorsprünge am vorderen Ende bzw. am hinteren Ende der Aussparung umfasst, gesehen in der longitudinalen Richtung des Schuhs, wobei jeder an entsprechende Vertiefungen oder Öffnungen des Gehäuses (110) koppelt.

8. Schuh (1) nach einem der vorherigen Ansprüche, wobei ein abnehmbarer Gurt (130) weiter in der Aussparung (120) angeordnet ist, um das Entnehmen des Gehäuses (110) von der Aussparung (120) zu vereinfachen.

9. Schuh (1) nach dem vorherigen Anspruch, wobei der abnehmbare Gurt (130) in der Aussparung (120) angeordnet ist, sodass er unter dem Gehäuse (110), welches in der Aussparung angeordnet ist, positioniert ist und dass ein Ende (131, 132) des Gurtes (130) sich über die obere Kante der Aussparung (120) erstreckt.

10. Schuh (1) nach einem der zwei vorherigen Ansprüche, wobei der abnehmbare Gurt (130) einen Ausschnitt (133) umfasst, welcher durch einen Vorsprung (117) des Gehäuses (110) angekoppelt ist, wenn das Gehäuse in der Aussparung (120) des Schuhs angeordnet ist.

11. Schuh (1) nach Anspruch 1, wobei das Verstärkungselement einen Stützbalken umfasst, welche vorzugsweise Torsionsflexibilität des Fußes erlaubt, d.h. Rotation des Vorderfußes relativ zum Hinterfuß.

12. Schuh nach einem der vorherigen Ansprüche, wobei der Schuh weiter eine Befestigungsabdeckung (30, 150) umfasst, welche an die Schnürsenkel oder andere Befestigungsmittel des Schuhs koppelt, und in welche eine zweite elektronische Baugruppe angeordnet werden kann.

## Revendications

1. Chaussure (1), en particulier chaussure de sport, comprenant une unité formant semelle, dans laquelle l'unité formant semelle comprend un évidement (20, 120) destiné à recevoir d'une manière amovible un boîtier (110) d'un ensemble électronique, en particulier un podomètre électronique, dans laquelle
a. l'évidement (20, 120) est disposé dans la semelle intercalaire d'une unité formant semelle, qui est recouverte par une semelle première pouvant de préférence être enlevée quand la chaussure est en cours d'utilisation,
b. l'évidement (20, 120) est disposé dans la région du centre du pied de l'unité formant semelle,
c. l'évidement (120) comprend au moins une saillie entrant en prise avec un renfoncement ou une ouverture correspondante du boîtier (110),
d. l'évidement (20) étant intégré dans un élément de renforcement de la semelle de la chaussure.

2. Chaussure (1) selon l'une des revendications précédentes, dans laquelle l'unité formant semelle comprend une ouverture et/ou une fenêtre pour inspecter et/ou manoeuvrer l'ensemble électronique.

3. Chaussure (1) selon l'une des revendications précédentes, dans laquelle l'évidement (20, 120) a une forme correspondant à la forme du boîtier (10, 110) de l'ensemble électronique.

4. Chaussure (1) selon la revendication précédente, dans laquelle l'évidement (20) comprend au moins un renfoncement ou une ouverture (22) destiné à entrer en prise avec une saillie (11) du boîtier (10).

5. Chaussure (1) selon la revendication précédente, dans laquelle l'évidement (20) comprend au moins deux renfoncements ou ouvertures (22), qui sont sensiblement opposées l'une à l'autre.

6. Chaussure (1) selon l'une des revendications précédentes, dans laquelle l'évidement (20) comprend au moins un élément élastique, de préférence une surface de séparation élastique, pour attacher fermement le boîtier (10) dans l'évidement (20).

7. Chaussure (1) selon la revendication précédente, dans laquelle l'évidement (120) comprend une, de préférence deux saillies, respectivement à l'extrémité avant et à l'extrémité arrière de l'évidement, quand on regarde dans la direction longitudinale de la chaussure, chacune entrant en prise avec des renfoncements ou ouvertures correspondants du boîtier (110).

8. Chaussure (1) selon l'une des revendications précédentes, dans laquelle une courroie d'enlèvement (130) est en outre disposée dans l'évidement (120) pour faciliter l'enlèvement du boîtier (110) de l'évidement (120).

9. Chaussure (1) selon la revendication précédente, dans laquelle la courroie d'enlèvement (130) est disposée dans l'évidement (120) de façon à être positionnée en-dessous du boîtier (110) disposé dans l'évidement, et qu'une extrémité (131, 132) de la courroie (130) s'étende par-dessus le bord supérieur de l'évidement (120).

10. Chaussure (1) selon l'une des deux revendications précédentes, dans laquelle la courroie d'enlèvement (130) comprend une découpe (133) avec laquelle entre en prise une saillie (117) du boîtier (110) quand le boîtier est disposé à l'intérieur de l'évidement (120) de la chaussure.

11. Chaussure (1) selon la revendication 1, dans laquelle l'élément de renforcement comprend une barre de soutien, qui de préférence autorise une flexion du pied en torsion, c'est-à-dire une rotation de l'avant-pied par rapport à la partie arrière du pied.

12. Chaussure selon l'une quelconque des revendications précédentes, la chaussure comprenant en outre un couvercle d'attachement (30, 150), entrant en prise avec les lacets ou d'autres moyens d'attachement de la chaussure, et dans lequel un deuxième ensemble électronique peut être disposé.
